**Europäisches Patentamt**

**European Patent Office**

**Office européen des brevets**

(11) Veröffentlichungsnummer: **0 356 766**
**A1**

## (12) EUROPÄISCHE PATENTANMELDUNG

(21) Anmeldenummer: **89114606.0**

(22) Anmeldetag: **08.08.89**

(51) Int. Cl.⁵ **A61F 2/16**

(30) Priorität: **10.08.88 DE 3827072**

(43) Veröffentlichungstag der Anmeldung:
**07.03.90 Patentblatt 90/10**

(84) Benannte Vertragsstaaten:
**AT BE CH ES FR GB GR IT LI NL SE**

(71) Anmelder: **Fromberg, Gunter, Dr. med.**
**Gudesbergstrasse 10**
**D-6690 St. Wendel(DE)**

(72) Erfinder: **Fromberg, Gunter, Dr. med.**
**Gudesbergstrasse 10**
**D-6690 St. Wendel(DE)**

(74) Vertreter: **Bernhardt, Winfrid, Dr.-Ing.**
**Kobenhüttenweg 43**
**D-6600 Saarbrücken(DE)**

(54) **Künstliche Augenlinse.**

(57) Bei einer künstlichen Augenlinse zur Implantation im Kapselsack der entfernten natürlichen Augenlinse mit einem Linsenkörper (1) und einer einstückig mit diesem hergestellten, im ganzen scheibenförmigen Halterung (2), die mit ihrem Außenrand im Umfang des Kapselsackes anliegt und den Abstand zwischen dem Umfang des Kapselsackes und dem, kleineren, Umfang des Linsenkörpers (1) überbrückt, ist die Biegesteifigkeit der scheibenförmigen Halterung (2) zwischen dem Außenrand und dem Linsenkörper (1) gestuft (9), derart, daß sie vom Außenrand nach dem Linsenkörper (1) hin mindestens einmal erhöht ist.

An der Stufe entsteht eine "Sollbiegestelle", die bei Druck auf die Halterung von außen mindestens einen wesentlichen Teil der Verformung aufnimmt. Damit wird ein Herauskippen der Linse aus ihrer Ebene weitgehend vermieden.

Eine ringscheibenförmige Halterung (2) weist beispielsweise eine ringförmige Verdickung (4) nahe ihrer Mitte und außerdem eine ringförmige Verdickung (3) am Außenrand auf.

Fig. 2

## Künstliche Augenlinse

Die Erfindung betrifft eine künstliche Augenlinse zur Implantation im Kapselsack der entfernten natürlichen Augenlinse mit einem Linsenkörper und einer einstückig mit diesem hergestellten, im ganzen scheibenförmigen Halterung, die mit ihrem Außenrand im Umfang des Kapselsackes anliegt und den Abstand zwischen dem Umfang des Kapselsackes und dem, kleineren, Umfang des Linsenkörpers überbrückt.

Solche künstlichen Augenlinsen sind unter der Bezeichnung "Disc-Linsen" bekannt. Nimmt im Laufe der Zeit der Druck von außen auf die Halterung, insbesondere ungleichmäßig, zu, so besteht das Problem, daß die künstliche Augenlinse aus ihrer ursprünglichen Ebene herausspringt; gegenüberliegende Abschnitte der Halterung stellen sich in einem Winkel zu dieser Ebene. Stellen sich die Abschnitte an der gleichen Seite aus der Ebene heraus, bleibt der Linsenkörper parallel zu ihr und weiter funktionsfähig. Meist winkeln sich jedoch die Abschnitte nach verschiedenen Seiten ab und stellen damit den Linsenkörper schräg zu der Ebene. Dann kann die künstliche Augenlinse nur noch ersetzt oder reponiert werden.

Der Erfindung liegt die Aufgabe zugrunde, bei einer künstlichen Augenlinse das Herauskippen der Linse aus ihrer Ebene möglichst zu vermeiden.

Gemäß der Erfindung ist zu diesem Zweck bei einer künstlichen Augenlinse der eingangs bezeichneten Art vorgesehen, daß die Biegesteifigkeit der scheibenförmigen Halterung zwischen dem Außenrand und dem Linsenkörper gestuft ist derart, daß sie vom Außenrand nach dem Linsenkörper hin mindestens einmal erhöht ist.

So entsteht an der Erhöhung eine "Sollbiegestelle". Hat sich bisher der innere Rand der Halterung, d.h. am Übergang von der Halterung zum Linsenkörper, als die Schwachstelle erwiesen, die hier mit zwei gegenüberliegenden Knickungen die gesamte Verformung aufnahm, so fängt jetzt gewissermaßen die jeweilige Sollbiegestelle mindestens einen wesentlichen Teil der Verformung davor ab. Die Halterung selbst wird im Abstand zwischen ihrem Außenrand und ihrem Innenrand verbogen. Die am Innenrand noch wirksamen Kräfte führen dort nicht mehr zu der bisherigen Knickung. Der Linsenkörper bleibt im wesentlichen in seiner Lage.

Die Möglichkeiten der Ausgestaltung sind vielfältig.

Ein Ausführungsbeispiel ist in der Zeichnung dargestellt.

Fig. 1 zeigt eine künstliche Augenlinse in Ansicht,

Fig. 2 zeigt die Augenlinse im Schnitt.

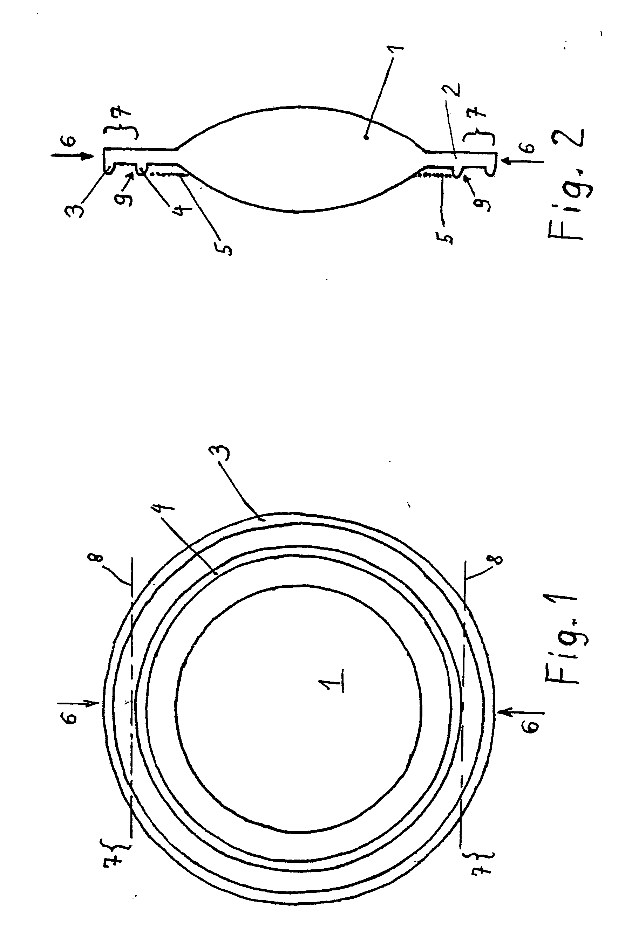

Die künstliche Augenlinse nach Fig. 1 und 2 gliedert sich in einen Linsenkörper 1 und eine Halterung 2, die den Linsenkörper als eine Ringscheibe umgibt. Am Außenrand der Halterung 2 und zugleich der Augenlinse insgesamt weist die Halterung 2 eine ringförmige Verdickung 3 auf. Eine weitere ringförmige Verdickung 4 zieht sich etwa durch die Mitte der Halterung 2. Beide Verdickungen 3 und 4 gehen in Form eines Wulstes nur nach der einen Seite der scheibenförmigen Halterung 2.

Alles ist einstückig hergestellt aus einem körperverträglichen elastischen Material, z.B. Silikonkautschuk, Polyhema u. dgl..

Der Durchmesser der Augenlinse beträgt am Außenrand 9,5 mm und am Umfang des Linsenkörpers 5,6 mm. Die Dicke der scheibenförmigen Halterung 2 betägt 0,3 mm. Die Verdickungen 3 und 4 haben eine Höhe sowie eine Breite von 0,25 mm.

Mit gepunkteten Linien 5 ist die Variante angedeutet, daß die Halterung 2 dort zwischen dem Linsenkörper 1 und der Verdickung 4 durchgehend die Dicke wie an der Verdickung 4 hat.

Betrachtet man diese Variante, so ist ohne weiteres ersichtlich, daß an der Verdickung 4 eine Stufe 9 gebildet ist, an der die Biegesteifigkeit der Halterung 2 sich erhöht. Kraftausübung gemäß den Pfeilen 6 führt dann nur zu einem Umbiegen der Abschnitte 7 an oder bis zu den in Fig. 1 an den Stufen strichpunktiert eingezeichneten Linien 8.

Aber auch bei gleicher Dicke der Halterung 2 innerhalb der ringförmigen Verdickung 4 wie außerhalb bildet die Verdickung 4 als ringförmige Versteifungsrippe eine Stufe 9, an der sich die Biegesteifigkeit erhöht, wenngleich sie danach, d.h. innerhalb der ringförmigen Verdickung 4, wieder vermindert ist. Auch in diesem Falle kommt es hauptsächlich zur Biegung an oder bis zu den Linien 8. Der Bereich innerhalb der ringförmigen Verdickung 4 ist dann jedoch nicht so absolut unbiegsam wie im Falle der gepunkteten Linie 5, sondern kann gleichfalls noch etwas Verformung aufnehmen.

Die vorgesehene Stufung könnte anders als mit der Dicke der scheibenförmigen Halterung erzeugt werden, d.h. die Stufe 9 könnte statt durch die ringförmige Verdickung 4 oder die verdickte Fortsetzung der Halterung 2 nach innen gemäß Linie 5 beispielsweise auch in der Weise gebildet sein, daß bei gleichbleibender Dicke der scheibenförmigen Halterung 2, mit oder ohne Verdickung am Außenrand, der äußere Abschnitt 7 durch eine Lochung oder in anderer Weise geschwächt ist. Analog zu der ringförmigen Verdickung 4 könnte dann innerhalb eines ungeschwächten Ringes, beispielsweise wiederum von der Breite der ringförmigen Verdickung 4, noch einmal eine gleiche Schwächung vorgenommen werden wie in dem Abschnitt 7.

Grenzfall einer Lochung wäre beispielsweise, daß nur noch mehr oder weniger breite Speichen übrigen bleiben.

Statt der einseitigen ringförmigen Verdickungen 3 und 4 wären auch beidseitige Verdickungen möglich.

Die nach der Erfindung vorgesehene Stufung braucht nicht abrupt zu sein. Die Oberfläche der Halterung könnte auch gewellt erscheinen, beispielsweise auch auf beiden Seiten gegenläufig.

Die im ganzen scheibenförmige Halterung braucht nicht kreisförmig zu sein. Sie wäre auch oval, eiförmig, gleichmäßig oder ungleichmäßig dreieckig und in noch anderer Gestaltung denkbar.

**Ansprüche**

1. Künstliche Augenlinse zur Implantation im Kapselsack der entfernten natürlichen Augenlinse mit einem Linsenkörper (1) und einer einstückig mit diesem hergestellten, im ganzen scheibenförmigen Halterung (2), die mit ihrem Außenrand im Umfang des Kapselsackes anliegt und den Abstand zwischen dem Umfang des Kapselsackes und dem, kleineren, Umfang des Linsenkörpers (1) überbrückt,
dadurch gekennzeichnet,
daß die Biegesteifigkeit der scheibenförmigen Halterung (2) zwischen dem Außenrand und dem Linsenkörper (1) gestuft (9) ist derart, daß sie vom Außenrand nach dem Linsenkörper (1) hin mindestens einmal erhöht ist.

2. Künstliche Augenlinse nach Anspruch 1,
dadurch gekennzeichnet,
daß die Biegesteifigkeit nach der Erhöhung wieder vermindert ist.

3. Künstliche Augenlinse nach Anspruch 1 oder 2,
dadurch gekennzeichnet,
daß die Stufung (9) mit der Dicke der scheibenförmigen Halterung (2) erzeugt ist.

4. Künstliche Augenlinse nach Anspruch 3,
dadurch gekennzeichnet,
daß eine ringscheibenförmige Halterung (2) eine ringförmige Verdickung (4) nahe ihrer Mitte und eine ringförmige Verdickung (3) am Außenrand aufweist.

5. Künstliche Augenlinse nach Anspruch 4,
dadurch gekennzeichnet,
daß die Verdickungen (3;4) einseitig sind, vorzugsweise auf derselben Seite der ringscheibenförmigen Halterung (2).

6. Künstliche Augenlinse nach Anspruch 4 oder 5 aus Silikonkautschuk,
dadurch gekennzeichnet,

daß sie am Außenrand einen Durchmesser von 9 bis 10 mm, vorzugsweise 9,4 bis 9,6 mm, aufweist, am Umfang des Linsenkörpers (1) einen Durchmesser von 4 bis 7 mm, vorzugsweise 5,5 bis 5,7 mm, eine Dicke der ringscheibenförmigen Halterung (2) von 0,28 bis 0,32 mm, vorzugsweise etwa 0,3 mm, an der erstgenannten Verdickung (4), die eine Breite von 0,23 bis 0,27 mm, vorzugsweise etwa 2,5 mm, hat, eine Dicke von 0,23 bis 0,27 mm, vorzugsweise etwa 0,25 mm, und an der Verdickung (3) am Außenrand, die eine Breite von 0,23 bis 0,27 mm, vorzugsweise etwa 0,25 mm, hat, eine Dicke von 0,23 bis 0,27 mm, vorzugsweise etwa 0,25 mm.

Fig. 1

Fig. 2

EP 0 356 766 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 585 456 (BLACKMORE)<br>* Insgesamt *<br>--- | 1,3 | A 61 F 2/16 |
| A | FR-A-2 530 457 (MAZZOCCO)<br>* Zusammenfassung; Seite 11, Zeile 34 - Seite 18, Zeile 26; Figuren *<br>--- | 1,2,4 | |
| A | US-A-4 254 509 (TENNANT)<br>--- | | |
| A | EP-A-0 202 049 (BAXTER)<br>--- | | |
| A | US-A-4 588 406 (FEDOROV)<br>--- | | |
| A | DE-A-3 626 869 (ROCHELS)<br>----- | | |

RECHERCHIERTE SACHGEBIETE (Int. Cl.5)

A 61 F

Der vorliegende Recherchenbericht wurde für alle Patentansprüche erstellt

| Recherchenort | Abschlußdatum der Recherche | Prüfer |
|---|---|---|
| DEN HAAG | 14-11-1989 | STEENBAKKER J. |